# EUROPÄISCHE PATENTANMELDUNG

(11) **EP 0 615 760 A1**
(43) Veröffentlichungstag der Anmeldung: **21.09.1994**
(21) Anmeldenummer: 94104032.1
(22) Anmeldetag: 16.03.1994
(51) Int. Cl.: A61M 1/16

(54) **Hämodialysegerät mit einer Bilanzkammer**

(30) Priorität: 18.03.1993 DE 4308586
(71) Anmelder: Fresenius AG, D-61350 Bad Homburg v.d.H (DE)
(72) Erfinder: Polaschegg, Hans-Dietrich, Dr., D-61440 Oberursel (DE)
(74) Vertreter: Fuchs, Luderschmidt & Partner Patentanwälte

(57) **Zusammenfassung**

Dialysegerät (10) mit einem Dialysator (12) und einer einzigen Bilanzkammer (30), bei der das Innenvolumen der Bilanzkammer (30) höchstens 2/3 des Volumens der Dialysierflüssigkeitskammer (18) des Dialysators (12) ist. Infolgedessen wird im Rezirkulationstakt höchstens 2/3 des Volumens der Dialysierflüssigkeitskammer (18) durch frische Dialysierflüssigkeit ersetzt.

## Beschreibung

Die Erfindung betrifft ein Hämodialysegerät mit einem Dialysator, der durch eine semipermeable Membran in eine Dialysierflüssigkeitskammer und eine Blutkammer geteilt ist, einem Blutweg, der durch die Blutkammer gelegt ist, einem Dialysierflüssigkeitsweg, der durch die Dialysierflüssigkeitskammer gelegt ist und dessen erstes Ende mit einer Dialysierflüssigkeitsquelle und dessen zweites Ende mit einem Abfluß verbunden sind, wobei der Dialysierflüssigkeitsweg in eine mm Dialysator führenden Zuleitung und eine vom Dialysator abgehenden Abzugsleitung geteilt ist, einer Bilanzkammer, die durch eine bewegliche Wand in zwei Bilanzkammerhälften geteilt ist, wobei die eine Bilanzkammerhälfte mit einem ersten Zulaufleitungsstück, die mit der Dialysierflüssigkeitsquelle verbunden ist, und mit einem zweiten Zulaufleitungsstück, die mit dem Dialysatoreingang verbunden ist, und die andere Bilanzkammerhälfte mit einem ersten vom Dialysatorauslaß abgehenden Abflußleitungsstück und einem zweiten zum Abfluß führenden Abflußleitungsstück strömungsmäßig verbunden sind, einer das erste Abflußleitungsstück angeschlossenen Ultrafiltrationseinheit, einer ersten, in das erste Zuleitungsstück eingeschalteten Absperranordnung, einer zweiten, in das zweite Zuleitungsstück eingeschalteten Absperranordnung, einer dritten, in das erste Abflußleitungsstück eingeschalteten Absperranordnung, einer vierten, in das zweite Abflußleitungsstuck eingeschalteten Absperranordnung, einer in das erste Zuleitungsstück eingeschalteten Zuführungspumpe, einer in das erste Ableitungsstück eingeschalteten Dialysierflüssigkeitspumpe und mit einer Steuer/Regeleinheit, die in einem ersten bzw. zweiten Takt das erste und vierte Absperranordnungspaar bzw. das zweite und dritte Absperranordnungspaar aktiviert bzw. desaktiviert.

Hämodialysergeräte mit volumetrischer Ultrafiltrationskontrolle verfügen üblicherweise über ein geschlossenes Dialysierflüssigkeitssystem, aus dem vorbestimmte Mengen an Dialysierflüssigkeit entzogen werden, die aufgrund des geschlossenen Systems aus der extrakorporalen Blutbahn und damit dem Patienten korrespondierend durch die Dialysator-Membran hindurch ergänzt werden. Um einen im wesentlichen stetigen Dialysierflüssigkeitsfluß trotz des geschlossenen Systems zwischen einer Dialysierflüssigkeitsquelle einerseits und dem Dialysator andererseits zu erreichen, ist in zahlreichen Hämodialysegeräten eine Bilanziereinheit eingeschaltet, die aus zwei Bilanzkammern besteht, die jeweils durch eine bewegliche Membran in zwei Bilanzhammerhälften geteilt ist. Jede dieser vier Bilanzkammerhälften verfügt über zwei Zulauf- und zwei Ablaufleitungen (insgesamt 8 Leitungsanschlüsse), in die jeweils ein Absperrorgan (also 8 Absperrorgane) eingefügt ist. Die Bilanzkammern sind dabei so geschaltet, daß die eine Bilanzkammer jeweils in den Füll/Entleerungsbetrieb und die andere Bilanzkammer in den Dialysierbetrieb geschaltet sind. Es erfolgt taktweise eine Umschaltung der beiden Bilanzkammern, sobald die frische Dialysierflüssigkeit vollständig durch die Dialysierflüssigkeitskammer gefördert ist. Infolgedessen wird jeweils eine Kammer mit frischer Dialysierflüssigkeit gefüllt, während die eben geladene Flüssigkeit aus der anderen durch den Dialysator zirkuliert wird. Hierdurch erreicht man einen nahezu gleichmäßigen Fluß, der nur während der Umschaltung von der einen auf die andere Kammer unterbrochen wird. Derartige Systeme sind beispielsweise in der deutschen Offenlegungsschrift 28 38 414 beschrieben.

Gegenüber dem kontinuierlich arbeitenden Bilanzierungssystem mit zwei Bilanzkammern existieren auch Systeme mit einer einzigen, großen Bilanzkammer bzw. Speicherkammer, wie sie beispielsweise in der DE-OS 25 44 258 beschrieben ist oder bei dem Dialysegerät der Firma Hospal verwirklicht ist. Mit einer solchen Bilanzkammer wird üblicherweise in einem ersten Arbeitsschritt möglichst schnell die Bilanzkammer gefüllt, während in einem zweiten, wesentlich langsamer ablaufenden Arbeitsschritt die Dialysierflüssigkeit durch die Dialysierflüssigkeitskammer des Dialysators zirkuliert wird. Dadurch erreicht man, daß das Intervall, in dem keine Dialysierflüssigkeit durch den Dialysator strömt, gering gehalten wird.

Beide Systeme sind sowohl vorrichtungstechnisch als auch steuerungstechnisch sehr aufwendig. Einerseits wird bei der Doppelbilanzkammermethode gegenüber der Einfachbilanzkammermethode eine zusätzliche Bilanzkammer benötigt, während im Falle des Betriebs mit einer Bilanzkammer hohe Ansprüche an die Fülleistung gestellt werden, was zu einem vorzeitigen Verschleiß der Pumpen führen kann.

Der Erfindung liegt die Aufgabe zugrunde, ein Hämodialysegerät der eingangs genannten Art zu schaffen, das gegenüber dem Dialysegerät des Standes der Technik apparativ einfacher ist, wobei zumindest eine gleiche Dialysierleistung erzielt wird.

Die Lösung der Aufgabe gelingt dadurch, daß im Rezirkulationstakt die Dialysierflüssigkeitspumpe höchstens 2/3 des Volumens der Dialysierflüssigkeitskammer mit frischer Dialysierflüssigkeit ersetzt.

Es wurde überraschenderweise festgestellt, daß es für die Austauschleistung des Dialysators unerheblich ist, ob die Flüssigkeitsströmung durch den Dialysator gleichmäßig wie bei der Doppelbilanzkammermethode oder diskontinuierlich wie bei der Einbilanzkammermethode erfolgt, sofern das Volumen der Bilanzkammer höchsten 2/3 des Volumens der Dialysierflüssigkeitskammer beträgt.

Dabei wird vorausgesetzt, daß im Zirkulationsbetrieb jeweils das mit frischer Flüssigkeit gefüllte gesamte Bilanzkammervolumen mit Hilfe der Umwälzpumpe durch die Dialysierflüssigkeitskammer des Dialysators gepumpt wird, was üblicherweise der Fall ist. Insofern fällt unter das Lösungsmerkmal auch die Ausführungsform, daß mit Hilfe der Umwälzpumpe ein Dialysierflüssigkeitsvolumen aus der Bilanzkammer entnommen wird, das höchstens 2/3 des Volumens der Dialysierflüssigkeitskammer des Dialysators entspricht. Bei dieser Ausführungsform ist also das Bilanzkammervolumen unabhängig von dem Volumen der Dialysierflüssigkeitskammer, so daß es nur auf eine Volumen-/oder Zeitsteuerung der Dialysierflüssigkeitspumpe in Abhängigkeit von ihrer Pumprate ankommt.

Erfindungsgemäß ist es also ausschlaggebend, daß die Große des Volumens innerhalb einer Förderperiode kleiner ist als das dialysierflüssigkeitsseitige Füllvolumen des Dialysators.

Gemäß einer bevorzugten Ausführungsform ist es weiterhin vorteilhaft, wenn der mittlere Fluß der Dialysierflüssigkeit etwa gleich ist, d.h., etwa gleiche Zeiten für den Füll- und Zirkulationsbetrieb zum Einsatz kommen.

Es sind nämlich die Strömungen der Dialysierflüssigkeit durch den Dialysator und die Diffusion von harnpflichtigen Substanzen durch die Membran des Dialysators voneinander unabhängig. Die Strömung folgt dem Druckgefälle der Dialysierflüssigkeitspumpe (Umwälzpumpe) und verläuft im wesentlichen parallel zur Membranoberfläche, während die Diffusion dem Konzentrationsgefälle zwischen dem Blut einerseits und der Dialysierflüssigkeit andererseits folgt, also im wesentlichen senkrecht zur Membranoberfläche gerichtet ist.

Demzufolge sind bei der Bewegung eines harnpflichtigen Moleküls beide Komponenten überlagert. Damit ein Molekül letztlich den Membranbereich und den Ausgang des Dialysators verläßt, ist es also lediglich erforderlich, daß es einen bestimmten Abstand von der Membran erreicht und anschließend zum Ausgang transportiert wird. Unerheblich ist dabei, welchen Abstand das Molekül dann von der Membranoberfläche hat Dabei ist das Strömungsverhalten im Dialysierflüssigkeitsbereich ohnehin unerheblich, solange das Molekül durch die Membran diffundiert oder sich noch im Blutbereich befindet.

Erfindungsgemäß ist es dabei vorteilhaft, daß bei Einhaltung der beanspruchten Volumenverhältnisse die im Inneren der Dialysierflüssigkeitskammer strömenden Flüssigkeitsteile bei der Zirkulation der Dialysierflüssigkeit durch die Kammer nicht wieder aus der Kammer hinausgeschleust werden. Andererseits wird der Effektivitätsverlust erst dann signifikant, wenn das Volumen des Flüssigkeitsaustauschs das beanspruchte Volumenverhältnis übersteigt.

Da das dialysatseitige Füllvolumen d.h., das Volumen der Dialysierflüssigkeitskammer üblicher Kapillardialysatoren etwa 150 ml (bei dem Dialysator der Firma FRESENIUS F40) ist, sollte das Zirkulationsvolumen in diesem Fall höchstens etwa 100 ml betragen und vorzugsweise unterhalb 75 ml. insbesondere etwa bei 30 ml liegen.

Das Beispiel erläutert die Erfindung.

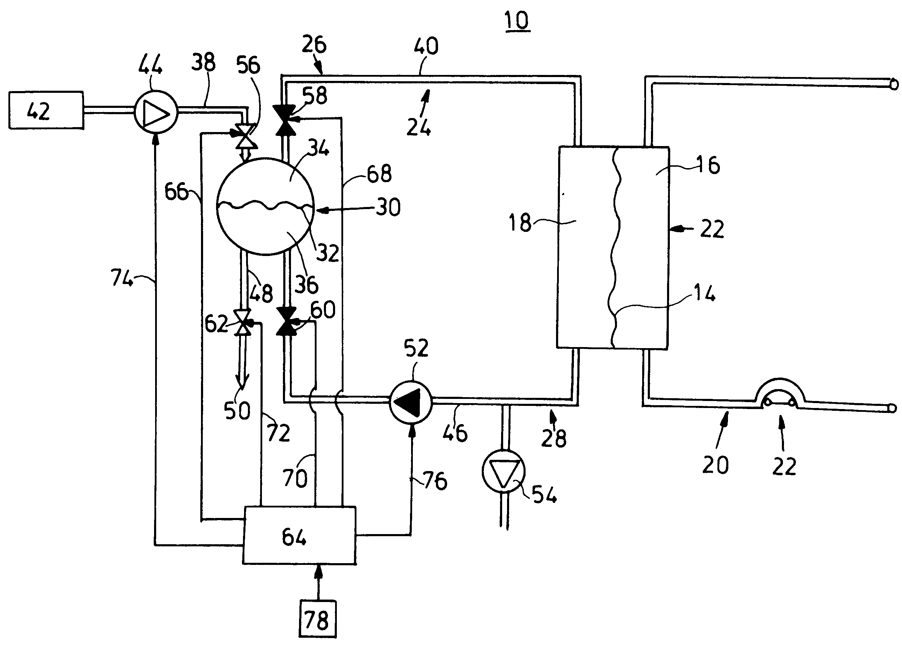

Die Figur zeigt schematisch ein Dialysegerät gemäß der Erfindung.

Mit 10 ist ein Dialysegerät gezeigt, das einen Dialysator 12 aufweist, der durch eine semipermeable Membran 14 in eine Blutkammer 16 und eine Dialysierflüssigkeitskammer 18 geteilt ist.

Durch die Blutkammer 16 ist ein Blutweg 20 gelegt, in den eine Blutpumpe 22 eingeschaltet ist. Dieser Blutweg 20 ist schematisch dargestellt und üblicher Art.

Durch die Dialysierflüssigkeitskammer 18 ist ein Dialysierflüssigkeitsweg 24 gelegt, der in eine Dialysierflüssigkeitszuleitung 26 und eine Abzugsleitung 28 unterteilt ist. Desweiteren ist eine Bilanzkammer 30 vorgesehen, die durch eine bewegliche Wand 32 in eine erste Bilanzkammerhälfte 34 zur Aufnahme von frischer Dialysierflüssigkeit und in eine zweite Bilanzkammerhälfte 36 zur Aufnahme verbrauchter Dialysierflüssigkeit geteilt ist.

Durch die erste Bilanzkammerhälfte 34 ist die Zuleitung 26 gelegt und dabei in ein erstes Zuleitungsstück 38 und ein zweites Zuleitungsstück 40 unterteilt.

Das erste Zuleitungsstuck 38 ist an seinem einen Ende mit einer Dialysierflüssigkeitsquelle 42 verbunden und mündet wie vorstehend festgestellt - in die erste Bilanzkammerhälfte 34. Desweiteren ist in das erste Zuleitungsstück 38 eine Zuführungspumpe 44 eingeschaltet.

Das zweite Zuleitungsstuck 40 geht von der ersten Bilanzkammerhälfte 34 ab und mündet in die Dialysierflüssigkeitskammer 18.

Die Abzugsleitung 28 ist durch die zweite Bilanzkammerhälfte 36 gelegt und in ein erstes Abzugsleitungsstück 46 und ein zweites Abzugsleitungsstück 48 geteilt. Dabei geht das erste Abzugsleitungsstück 46 von der Dialysierflüssigkeitskammer 18 ab und mündet in die zweite Bilanzkammerhälfte 36, während das zweite Abzugsleitungsstück 48 von der zweiten Bilanzkammerhälfte 36 abgeht und in Abfluß 50 mündet.

In das erste Abzugsleitungsstück 46 ist weiterhin eine Dialysierflüssigkeitspumpe 52 eingeschaltet. Weiterhin geht üblicherweise von dem ersten Abzugsleitungsstück 46 eine Ultrafiltrationseinheit 54 ab.

Jeweils benachbart zu den ersten und zweiten Bilanzkammerhälfte 34, 36 sind in das erste Zuleitungsstück 38 ein erstes Ventil 56, in das zweite Zuleitungsstück 40 ein zweites Ventil 58, in das erste Abzugsleitungsstück 46 ein drittes Ventil 60 und in das zweite Abzugsleitungstück 48 ein viertes Ventil 62 eingeschaltet.

Die Ventile 56-62 sind mit einer Steuer- und Regeleinheit 64 über die Steuerleitungen 66-72 verbunden Weiterhin sind die Pumpen 44, 52 über die Steuerleitungen 74, 76 mit der Steuer- und Regeleinheit 64 verbunden.

In der Figur sind diejenigen Ventilpaare 56-62 und die Pumpen 44, 52 entweder hell oder dunkel in Abhängigkeit von ihrem jeweiligen aktivierten Zustand gezeichnet.

Das Dialysegerät 10 zeichnet sich zunächst dadurch aus, daß das Volumen der Dialysierflüssigkeitskammer 18 und das Volumen der Bilanzkammer 30, das sich durch die Volumina der Bilanzkammerhälften 34, 36 zusammensetzt, in einem Verhältnis von höchstens 1:2/3 gemäß einer ersten Ausführungsform vorliegen.

Gemäß einer zweiten Ausführungsform fördert die Dialysierflüssigkeitspumpe 52 höchstens 2/3 Volumenteile Dialysierflüssigkeit bezogen auf das Volumen der Dialysierflüssigkeitskammer 18.

Bevorzugte Volumenverhältnisse liegen bei höchstens 50, insbesondere 30 % und besonders vorzugsweise bei ca. 20 % des Volumens d er Bilanzkammer 18.

Von beiden Ausführungsformen ist jedoch die erste Ausführungsform bevorzugt, d.h., die Begrenzung des Volumens der Bilanzkammer 34 gegenüber dem Volumen der Dialysierflüssigkeitskammer 18.

Das Dialysegerät 10 arbeitet folgendermaßen:
In einem Takt wird das Ventilpaar 56, 62 und die Zuführungspumpe 44 aktiviert, während das Ventile 58, 60 geschlossen bleibt und die Pumpe 52 ebenfalls desaktiviert ist. Andererseits kann die Pumpe 52 jedoch auch im Leerlauf bei geschlossenem Ventil 60 weiterfördern.

Die Zuführungspumpe 44 fördert nun frische Dialysierflüssigkeit aus der Dialysierflüssigkeitsquelle 42 in die erste Bilanzkammerhälfte 34, wobei die bewegliche Wand 32 unter Verkleinerung des Volumens der zweiten Bilanzkammerhälfte 36 an deren Innenwand gedrückt wird. Hierdurch wird eventuell vorliegende vebrauchte Dialysierflüssigkeit durch das geöffnete vierte Ventil 62 in den Abfluß 50 verdrängt.

Sobald die erste Bilanzkammerhälfte 34 gefüllt ist, erzeugt die Steuereinheit 64 ein Umschaltsignal, was zur Folge hat, daß die Ventile 56, 62 geschlossen und die Ventile 58, 60 geöffnet und die Dialysierflüssigkeitspumpe 52 aktiviert wird. Dabei kann die Zuführungspumpe 44 entweder desaktiviert werden oder aber läuft im Leerlauf weiter.

In diesem zweiten Takt wird nun frische Dialysierflüssigkeit aus der ersten Bilanzkammerhälfte 34 zur Dialysierflüssigkeitskammer 18 des Dialysators 12 gefördert und verdrängt dort teilweise verbrauchte Dialysierflüssigkeit durch das erste Abzugsleitungsstück 46 in die zweite Bilanzkammerhälfte 36 und verschiebt dort die bewegliche Wand unter stetiger Verringerung des Volumens der ersten Bilanzkammerhälfte an deren Innenwand. Sobald der Fördervorgang durch Entleerung der ersten Bilanzkammerhälfte bzw. die Förderung der vorbestimmten Menge Dialysierflüssigkeit abgeschlossen ist, wird erneut ein Steuersignal von der Steuereinheit 64 erzeugt mit der Folge, daß erneut der erste Takt einsetzt.

Die Steuerung erfolgt entweder zeitgesteuert in Abhängigkeit von der Förderrate der beiden Pumpen 44, 52, wobei sichergestellt ist, daß jeweils - Ende eines jeden Takts die Bilanzkammer 30 gefüllt ist.

Andererseits kann jedoch aber auch das Umschaltsignal mittels eines Sensors (Durchflußsensor) gewonnen werden, der jeweils in den Leitungsstücken 38, 46 angeordnet ist und der bei abfallendem Durchfluß sein Signal an die Regel- und Steuereinrichtung 64 abgibt. Schließlich kann auch das Umschaltsignal aus dem Motorstrom der Pumpe 44, 52 gewonnen werden, wie dies in der DE-OS 28 38 414 beschrieben ist worauf Bezug genommen wird.

Gemäß einer bevorzugten Ausführungsform ist in etwa die Förderrate der Pumpen 44, 52 gleich, so daß sich in etwa gleiche Taktzeiten ergeben.

Gemäß der zweiten Ausführungsform hängt die der Dialysierflüssigkeitskammer 18 zugeführte Dialysierflüssigkeitsmenge lediglich von dem Volumen ab, das von der Dialysierflüssigkeitspumpe 52 der Dialysierflüssigkeitskammer 18 zugeführt wird. Mit einer Eingabeeinheit, die mit der Steuer- und Regeleinheit 64 verbunden ist, kann die Förderrate und die Förderzeit der Pumpe 52 vorbestimmt werden, so daß hierdurch ein bestimmtes Volumen Dialysierflüssigkeit festgelegt wird. Desweiteren kann das von Dialysator zu Dialysator unterschiedliche Volumen der Dialysierflüssigkeitskammer 18 ebenfalls eingegeben werden, so daß sich die beiden Volumina aufeinander abstimmen lassen. Schließlich kann auch direkt das Volumenverhältnis der beiden Volumina eingegeben werden.

Mit dieser Behandlungsmethode läßt sich bei nahezu gleicher Dialysiereffizienz der apparative Aufwand an der Bilanziereinheit entgegen der Auffassung der Fachwelt halbieren, die der Meinung war, daß nur das kontinuierliche Zuführen von Dialysierflüssigkeit zum Erfolg führen würde bzw. bei dem Einsatz nur einer Kammer ein Abfall an Effizienz und ein erhöhter apparativer Aufwand in Kauf genommen werden müßte.

## Patentansprüche

1. Hämodialysegerät mit einem Dialysator, der durch eine semipermeable Membran in eine Dialysierflüssigkeitskammer und eine Blutkammer geteilt ist, einem Blutweg, der durch die Blukammer gelegt ist, einem Dialysierflüssigkeitsweg, der durch die Dialysierflüssigkeitskammer gelegt ist und dessen erstes Ende mit einer Dialysierflüssigkeitsquelle und dessen zweites Ende mit einem Abfluß verbunden sind, wobei der Dialysierflüssigkeitweg in eine zum Dialysator führenden Zuleitung und eine vom Dialysator abgehenden Abzugsleitung geteilt ist, einer Bilanzkammer, die durch eine bewegliche Wand in zwei Bilanzkammerhälften geteilt ist, wobei die eine Bilanzkammerhälfte mit einem ersten Zulaufleitungsstück, die mit der Dialysierflüssigkeitsquelle verbunden ist, und mit einem zweiten Zulaufleitungsstück, die mit dem Dialysatoreingang verbunden ist, und die andere Bilanzkammerhälfte mit einem ersten vom Dialysatorauslaß abgehenden Abflußleitungsstück und einem zweiten mm Abfluß führenden Abflußleitungsstück strömungsmäßig verbunden sind, einer a das erste Abflußleitungsstück angeschlossenen Ultrafiltrationseinheit, einer ersten, in das erste Zuleitungsstück eingeschalteten Absperranordnung, einer zweiten, in das zweite Zuleitungsstück eingeschalteten Absperranordnung, einer dritten, in das erste Abflußleitungsstück eingeschalteten Absperranordnung, einer vierten, in das zweite Abflußleitungsstück eingeschalteten Absperranordnung, einer in das erste Zuleitungsstück eingeschalteten Zuführungspumpe, einer in das erste Ableitungsstück eingeschalteten Dialysierflüssigkeitspumpe und mit einer Steuer-/Regeleinheit, die in einem ersten bzw. zweiten Takt das erste und vierte Absperranordnungspaar bzw. das zweite und dritte Absperranordnungspaar aktiviert bzw. desaktiviert, dadurch gekennzeichnet, daß im Rezirkulationstakt die Dialysierflüssigkeitspumpe (52) höchstens 2/3 des Volumens der Dialysierflüssigkeitskammer (18) mit frischer Dialysierflüssigkeit ersetzt.

2. Gerät nach Anspruch 1, dadurch gekennzeichnet, daß die Dialysierflüssigkeitspumpe (52) höchstens 50, insbesondere höchstens 30% des Volumens der Dialysierflüssigkeitskammer (18) durch frische Dialysierflüssigkeit ersetzt.

3. Gerät nach Anbruch 1 oder 2, dadurch gekennzeichnet, daß die Dialysierflüssigkeitspumpe (52) etwa 20 % des Volumens der Dialysierflüssigkeitskammer (18) mit frischer Dialysierflüssigkeit ersetzt.

4. Gerät nach einem der Ansprüche 1 bis 3, dadurch gekennzeichnet, daß das Innenvolumen der Bilanzkammer (34) höchstens 2/3, vorzugsweise 1/2, insbesondere 3/10 des Volumens der Dialysierflüssigkeitskammer (18) beträgt.

5. Gerät nach Anspruch 1, dadurch gekennzeichnet, daß die Förderzeiten der Zuführpumpe (44) und der Dialysierflüssigkeitspumpe (52) etwa gleich lang sind.
